# EUROPEAN PATENT APPLICATION

(11) **EP 3 381 447 A1**
(43) Date of publication of application: **03.10.2018**
(21) Application number: 18164314.9
(22) Date of filing: 27.03.2018
(51) Int. Cl.: A61K 9/48, C08B 13/00, C08L 1/32

(54) **INJECTION MOLDING COMPOSITION CONTAINING HYPROMELLOSE ACETATE SUCCINATE AND METHOD FOR PRODUCING SAME**

(30) Priority: 30.03.2017 JP 2017067981
(71) Applicant: Shin-Etsu Chemical Co., Ltd., Tokyo 100-0004 (JP)
(72) Inventor: WARASHINA, Shogo, Joetsu-shi, Niigata 942-8601 (JP); MARUYAMA, Naosuke, Joetsu-shi, Niigata 942-8601 (JP)
(74) Representative: De Vries & Metman

(57) **Abstract**

There are provided an injection molding composition capable of being injection-molded easily at a temperature lower than that of a conventional composition, as well as an injection molded product having improved strength. More specifically, there is provided an injection molding composition containing HPMCAS having a hydroxypropoxy molar substitution of 0.40 or more. In addition, there is provided a method for producing an injection molded product including a step of injecting into a mold the injection molding composition at from 50 to 250°C.

## Description

### BACKGROUND OF THE INVENTION

### 1. Field of the Invention

The present invention relates to an injection-molded product using a hypromellose acetate succinate and a method for producing the injection-molded product.

### 2. Related Art

Injection molding is one of the methods for producing a pharmaceutical solid preparation such as a hard capsule. This method is advantageous because it does not require a solvent so that energy in the drying step can be reduced.

For such a pharmaceutical solid preparation, a pharmaceutically acceptable, for example, an orally administrable polymer is generally used. As one example of the polymer, there is a hypromellose acetate succinate (which may hereinafter be called "HPMCAS"). The HPMCAS is a polymer having four substituents in total, i.e. two substituents: a methyl group (-CH₃) and a hydroxypropyl group (-C₃H₆OH) introduced into a cellulose skeleton to form an ether structure, and two substituents: an acetyl group (-COCH₃) and a succinyl group (-COC₂H₄COOH) introduced into the cellulose skeleton to form an ester structure.

The content of each substituent of HPMCAS listed in the Japanese Pharmacopoeia 17th Edition is as follows (see "Hypromellose Acetate Succinate" in Official Monographs of the Japanese Pharmacopoeia 17th Edition).

**Table 1**

| | content (% by weight) | molar substitution (MS)*1 |
|---|---|---|
| methoxy group | 12.0∼28.0 | 0.73∼2.83 |
| hydroxypropoxy group | 4.0∼23.0 | 0.10∼1.90 |
| acetyl group | 2.0∼16.0 | 0.09∼2.30 |
| succinyl group | 4.0∼28.0 | 0.08∼1.78 |

| | | |
|---|---|---|
| *1 The molar substitution means an average mole number of each substituent added per glucose ring unit of cellulose. | | |

As an injection-molded product containing HPMCAS, there has been reported, for example, a dosage form produced using an injected pharmaceutical composition containing HPMCAS (commercially available AS-LG having hydroxypropoxy molar substitution of from 0.16 to 0.35), a plasticizer, a lubricant and a solubility-modifying excipient, wherein a plasticizer is added to reduce a softening temperature of the HPMCAS (JP 2011-503048T which is a Japanese phase publication of WO 2009/087483).

In addition, there are proposed a method for hot-melt extruding a poorly water-soluble drug "posaconazole" and HPMCAS (commercially available AS-MF and AS-MG, each having hydroxypropoxy molar substitution of from 0.15 to 0.34) to produce a preparation (JP 2011-516612T which is a Japanese phase publication of WO 2009/129300), and a method for hot-melt extruding, as a lipid inhibitor, a CETP (cholesterol ester transfer protein) inhibitor which is a sparingly water-soluble drug and HPMCAS to produce a preparation (JP 2005-523895T which is a Japanese phase publication of WO 2003/063832.).

Further, there is proposed a method for hot-melt extruding a solid dispersion composition containing a sparingly water-soluble drug and HPMCAS having a hydroxypropoxy molar substitution of 0.40 or more (JP 2015-127316A).

### SUMMARY OF THE INVENTION

When HPMCAS alone is used for injection molding, a high injection molding temperature is required because commercially available HPMCAS now has a glass transition temperature (Tg) of from about 115 to 135°C. The high injection molding temperature is likely to cause decomposition or coloration of the HPMCAS so that a plasticizer is sometimes added to lower the injection molding temperature. However, addition of the plasticizer may decrease the strength of the injection-molded product thus obtained. Accordingly, a type of HPMCAS more suited for injection molding, that is, a type of HPMCAS capable of being injection-molded even at a lower temperature is demanded,

In the method described in JP 2011-503048T, the injection-molded product may have deteriorated strength because it contains a plasticizer. Moreover, triethyl citrate (TEC), triacetin or glycerol used as a plasticizer is in liquid form at an ambient temperature so that addition of such a plasticizer to HPMCAS causes agglomeration, thereby making uniform mixing difficult. Further, there is concern that TEC, which is acidic, causes the ester group of the HPMCAS to become unstable during storage, while triacetin itself is relatively unstable during storage.

In the hot-melt extrusion described in JP 2011-516612T, JP 2005-523895T and JP 2015-127316A, the extruded products are cut and pulverized, so that the strength of the extruded products is not described therein. Since many of sparingly water-soluble drugs have molecular weights lower than that of HPMCAS, addition of the sparingly water-soluble drug improves an extrusion property, but there is concern that the addition may markedly deteriorate the strength of the extruded product.

With the foregoing in view, the invention has been made. An object of the invention is to provide an injection molding composition capable of being injection-molded easily at a temperature lower than that of a conventional composition and capable of providing an injection-molded product having improved strength.

With a view to achieving the above-described object, the inventors have carried out an extensive investigation. As a result, it has been found that HPMCAS having a glass transition temperature (Tg) lower than that of conventional HPMCAS can be obtained by adjusting the hydroxypropoxy molar substitution, with respect with four substituents of the HPMCAS, to fall within a predetermined range, leading to the completion of the invention.

In one aspect of the invention, there is provided an injection molding composition comprising HPMCAS having a hydroxypropoxy molar substitution of 0.40 or more. In another aspect of the invention, there is provided a method for producing an injection-molded product comprising a step of injecting into a mold the injection molding composition at from 50 to 250°C.

According to the invention, injection molding at a temperature lower than a conventional temperature can be carried out so that an injection molded product having improved strength can be provided by the method for not causing decomposition of HPMCAS by heat or the like.

### DETAILED DESCRIPTION OF THE PREFERRED EMBODIMENTS

The invention will hereinafter be described in further detail.

The hydroxypropoxy molar substitution of the HPMCAS is 0.40 or more, preferably from 0.40 to 1.50, more preferably from 0.40 to 1.0, still more preferably from 0.40 to 0.90. When the hydroxypropoxy molar substitution is less than 0.40, an injection molding temperature becomes high so that hydrolysis occurs due to thermal decomposition of the HPMCAS, and a portion of the ester groups is liberated from its cellulose skeleton.

The hydroxypropoxy molar substitution can be determined by conversion of a value measured by the method described in "Hypromellose Acetate Succinate" in Official Monographs of the Japanese Pharmacopoeia 17th Edition.

The glass transition temperature (Tg) of the HPMCAS is preferably 115°C or less (further preferably from 60 to 115°C), more preferably 110° C or less (further preferably 60 to 110° C), still more preferably from 70 to 110° C, particularly preferably from 70 to 100° C. When the glass transition temperature is higher than 115°C, the injection molding temperature also becomes high so that there is a possibility of the above-described thermal decomposition.

The glass transition temperature (Tg) is usually measured with a differential scanning calorimeter (DSC) as described below. More specifically, 10 mg of HPMCAS in a nitrogen atmosphere is heated from room temperature (25°C) to 150°C at a rate of 10°C/min, cooled once to 25°C at a rate of 10°C/min, and then heated again to 230°C at a rate of 10°C/min to find an inflection point which is designated as a glass transition temperature. The reason why the glass transition temperature is measured under such an absolutely dry state is that water content in a sample affects the Tg value thus measured.

Regarding the methoxy group of the HPMCAS, which is one of the substituents other than the hydroxypropoxy group, the methoxy molar substitution is not particularly limited and is preferably from 0.70 to 2.83, more preferably from 1.00 to 2.40, still more preferably from 1.4 to 2.0.

The acetyl molar substitution of the HPMCAS is also not particularly limited, and is preferably from 0.10 to 2.30, more preferably from 0.10 to 1.00, still more preferably from 0.40 to 0.80.

The succinyl molar substitution of the HPMCAS is also not particularly limited, and is preferably from 0.10 to 1.78, more preferably from 0.10 to 1.00, still more preferably from 0.15 to 0.50.

The molar substitution of each substituent is measured in the same manner as that for the hydroxypropoxy molar substitution.

The viscosity at 20°C of a dilute (0.1 mol/L) aqueous sodium hydroxide solution containing 2% by weight of the HPMCAS is preferably from 1.1 to 20 mPa·s, more preferably from 1.5 to 3.6 mPa·s. When the viscosity is less than 1.1 mPa·s, the injection-molded product obtained may not have sufficient strength. When the viscosity is more than 20 mPa·s, the injection molding composition has a too high viscosity so that torque applied to a piston or screw may become excessive, thereby preventing rotation of the piston or screw or stopping the machine for safety. The viscosity can be determined by capillary tube viscometer used in Method I described in General Tests of HPMCAS in the Japanese Pharmacopoeia 17th Edition.

The HPMCAS may be produced, for example, by the method described in JP 54-061282A. Hypromellose (another name: hydroxypropyl methyl cellulose which may hereinafter also be called "HPMC") to be used as a starting material is dissolved in glacial acetic acid. Acetic anhydride and succinic anhydride as an esterifying agent, and sodium acetate as a reaction catalyst are added thereto, and heated for the reaction. After completion of the reaction, a large amount of water is added to the reaction product mixture to precipitate HPMCAS. The resulting precipitate is washed with water and then dried. When HPMC having a hydroxypropoxy molar substitution of 0.40 or more is used, the HPMCAS obtained from the HPMC also has a hydroxypropoxy molar substitution of 0.40 or more.

Further, according to the invention, the composition may contain an additive to produce a capsule having a preferable dissolution profile, physical stability, chemical stability and tensile strength, easily and reproducibly. Examples of the additive include a processing aid such as a lubricant and a reinforcing agent, a solubility-modifier, and an excipient.

In general, a plasticizer capable of reducing the viscosity during injection molding may be used to assist the injection molding. Although various types of plasticizer capable of plasticizing HPMCAS have been found, it is generally known that since the addition of a plasticizer plasticizes an injection molded product, the injection molded product has deteriorated strength and tensile properties. In particular, JP 2011-503048T has revealed that there is a possibility that replacement of propylene glycol and glycerol by polyethylene glycol results in a molded product of a certain polymer with relatively deteriorated strength and tensile properties. Moreover, TEC, triacetin or the like to be used as a plasticizer has acidic properties so that it may assist acid hydrolysis of the ester group during storage. The acid hydrolysis of the ester group of HPMCAS may cause a change in pH at which the HPMCAS dissolves, deterioration in acid resistance of the HPMCAS, or the like. From such a standpoint, the injection-molded product preferably contains a plasticizer as little as possible. According to the invention, the injection molding composition can be injection-molded at a temperature lower than that of a conventional composition so that addition of a plasticizer for reducing the injection temperature is not required. In addition, according to the invention, an injection-molded product having improved strength can be obtained from the injection molding composition so that a plasticizer may be added to reduce the viscosity during injection molding insofar as it does not deteriorate the strength of the product obtained by injection molding. The content of the plasticizer is preferably 8 parts by weight or less, more preferably 5 parts by weight or less based on 100 parts by weight of the HPMCAS.

Examples of the plasticizer include triethyl citrate (TEC), triacetin, tributyl citrate, acetyl triethyl citrate (ATEC), acetyl tributyl citrate (ATBC), dibutyl phthalate, dibutyl sebacate (DBS), diethyl phthalate, glycerol, vinylpyrrolidone glycol triacetate, polyethylene glycol, polyoxyethylene sorbitan monolaurate, propylene glycol, and fractionated coconut oil or castor oil, and combinations thereof.

The lubricant can provide lubricity to a die wall in an extrusion method and a mold wall in an injection molding method, and is used for reducing a strain which occurs during removal of a capsule from a mold. Examples of the lubricant include, but not limited to, stearyl alcohol, stearic acid, glycerol monostearate (GMS), talc, magnesium stearate, silicon dioxide, amorphous silicic acid, fumed silica, lauric acid, lecithin, sucrose fatty acid esters, higher fatty acid esters having from 12 to 18 carbon atoms and being derived from, for example, stearic acid, oleic acid, palmitic acid or lauric acid, and combinations thereof.

The reinforcing agent is used for obtaining an injection-molded product having improved strength. Examples of the reinforcing agent include, but not limited to, talc, titanium oxide, calcium carbonate, and combinations thereof.

Examples of the solubility-modifier include a disintegrant, a swelling solid and a surfactant. It is a substance used for assisting or improving the solubility of the injection-molded product. Addition of the solubility-modifier can result in the injection molding composition having a preferable dissolution profile.

The disintegrant is a substance that changes the swelling properties and assists release modification. When the injection-molded product is a capsule, speedy dissolution of the capsule in the digestive tract which absorbs an active substance encapsulated in the capsule is preferred to release the active substance. As the disintegrant, substances belonging to many different groups may be used to assist speedy disintegration. Examples of the disintegrant include, but not limited to, known super disintegrants typified by sodium starch glycolate, sodium carboxyl methylstarch, carmellose and a sodium salt thereof, croscarmellose sodium, low-substituted hydroxypropyl cellulose, polyvinylpyrrolidone (PVP), crosslinked PVP, a copolymer of PVP, and combinations thereof.

The swelling solid used as a solid having a swelling property can also assist release modification, though the swelling solid is not generally used as a disintegrant. Examples of the swelling solid include, but not limited to, polyethylene oxide; cellulose derivatives such as cellulose acetate phthalate, hydroxypropyl cellulose (HPC), hydroxypropyl methyl cellulose (HPMC), hydroxypropyl methyl cellulose phthalate (HPMCP) and other hydroxyalkyl cellulose derivatives; and combinations thereof.

The surfactant itself can work as an absorption enhancer when selected properly. Examples of the surfactant include an anionic surfactant such as sodium lauryl sulfate; a nonionic surfactant such as diglyceride, poloxamer, a polyoxyethylene sorbitan fatty acid ester (Tween 20, 60 and 80), a glycerin fatty acid ester, and a propylene glycol fatty acid ester; and a natural surfactant such as lecithin and sodium taurocholate.

Next, the method for producing an injection molded product will be described.

An injection molding composition is produced by a method comprising steps of: mixing HPMCAS having a hydroxypropoxy molar substitution of 0.40 or more and an optional component to form an injection molding composition, and extruding or injecting the injection molding composition with an injection molding machine into a desired shape such as a capsule-like, round or square shape or a columnar or film-like shape to obtain a molded product.

The injection molding composition can be molded into various shapes. The injection molding is used for obtaining preferably a capsule, more preferably a hard capsule. The capsule has a wall thickness of preferably from 0.1 to 1.0 mm, more preferably from 0.3 to 0.8 mm from the standpoint of improving the strength of a capsule and suppressing retardation of a dissolution profile. The hard capsule comprises a capsule base material to be orally administered, and is used for encapsulation of a homogenized mixture of an effective ingredient and an additive such as excipient, or encapsulation of a granulated or molded product formed by an appropriate method.

The capsule obtained from the injection molding composition is preferably enteric. The enteric capsule can protect a drug from gastric acid and be speedily dissolved in the small intestine which is an absorption site for the drug.

The injection molding machine is not particularly limited insofar as it is an injection molding machine capable of melting and kneading an injection molding composition with heating, while applying a shear force to the composition with a piston or screw, injecting the melted and kneaded composition from a die into a mold, and then cooling the injected composition. Specific examples include Haake MiniJet Pro (uniaxial piston injection molding system, product of Thermofisher Scientific,) and Roboshot i5A (biaxial screw type extruder, product of Fanuc).

The injection temperature is not particularly limited. The injection molding is carried out preferably within a temperature range in which the injection molding composition can be melted and injected smoothly, while avoiding thermal decomposition of the HPMCAS and another component as much as possible. More specifically, the injection temperature is preferably from 50 to 250°C, more preferably from 60 to 200°C, still more preferably from 90 to 190°C. When the injection temperature is lower than 50°C, the composition may not be melted completely so that the extrusion or injection may make become difficult. When the injection temperature is more than 250°C, there is a possibility that the HPMCAS is decomposed.

The composition thus injected becomes a solid by cooling. It can be molded into a desired shape by directly obtaining a solid product by means of a 3D printer or by cooling in a mold. In order to uniformly distribute the injected composition in a mold, it is preferable to adjust the mold to an appropriate temperature correspondingly to a shape of the mold, and then perform cooling. The temperature of the mold is preferably from 0 to 250°C, more preferably from 20 to 200°C, still more preferably from 40 to 190°C.

### EXAMPLES

The invention will hereinafter be described specifically by Examples and Comparative Examples. It should not be construed that the invention is limited to or by Examples.

### <Synthesis of HPMCAS-1>

In a 50-L kneader, 12 kg of glacial acetic acid was placed and 6 kg of a hypromellose (HPMC) having a hydroxypropoxy molar substitution of 0,84 and a methoxy molar substitution of 1.58 was added and dissolved therein. Further, 4.1 kg of acetic anhydride, 1.5 kg of succinic anhydride, and 4.8 kg of sodium acetate were added thereto, and the resulting mixture was reacted at 85°C for 5 hours. After addition of 6.7 kg of purified water to the reaction product mixture and stirring, purified water was added to the resulting solution to precipitate HPMCAS in particle form. By filtration, a crude HPMCAS was collected. The crude HPMCAS was washed with purified water, and dried. Then it was sifted through a 10-mesh sieve (opening: 1700 µm) to obtain HPMCAS-1 having final water content of 1.2% by weight.

The content of each of the substituents of the HPMCAS-1 thus obtained was measured by the method described in the Japanese Pharmacopoeia 17th Edition. As a result, the hydroxypropoxy content was 21.1% by weight (molar substitution of 0.84), the methoxy content was 16.3% by weight (molar substitution of 1.58), the acetyl content was 8.5% by weight (molar substitution of 0.59), and the succinyl content was 14.2% by weight (molar substitution of 0.42). The viscosity at 20°C of a dilute (0.1 mol/L) aqueous sodium hydroxide solution containing 2% by weight of HPMCAS-1 was measured to be 2.92 mPa·s by capillary tube viscometry used in Method 1 described in the Japanese Pharmacopoeia 17th Edition.

### <Synthesis of HPMCAS-2 to 5>

HPMCAS-2 to 5 shown in Table 2 were obtained in the same manner as in Synthesis of HPMCAS-1 except that a starting material HPMC different in the content of each substituent was used and amounts of acetic anhydride and succinic anhydride were changed appropriately, respectively.

**Table 2**

| | molar substitution | | | | viscosity of 2 wt% solution when dissolved in aq. NaOH solution (mPa·s) |
|---|---|---|---|---|---|
| | hydroxy propoxy | methoxy | acetyl | succinyl | |
| HPMCAS-1 | 0.84 | 1.58 | 0.59 | 0.42 | 2.92 |
| HPMCAS-2 | 0.62 | 1.82 | 0.57 | 0.27 | 3.13 |
| HPMCAS-3 | 0.56 | 1.54 | 0.57 | 0.41 | 2.99 |
| HPMCAS-4 | 0.47 | 1.91 | 0.52 | 0.29 | 2.92 |
| HPMCAS-5 | 0.57 | 1.91 | 0.71 | 0.16 | 2.79 |
| HPMCAS-6 | 0.62 | 1.83 | 0.48 | 0.43 | 3.20 |
| HPMCAS-7 | 0.24 | 1.89 | 0.55 | 0.28 | 2.99 |

### <Measurement of glass transition temperature of HPMCAS>

The glass transition temperature (Tg) of HPMCAS-1 to 7 was measured using a differential scanning calorimeter ("DSC3200SA", product of Bruker). More specifically, 10 mg of each HPMCAS in a nitrogen atmosphere was heated from room temperature to 150°C at a rate of 10°C/min, cooled once to 25°C at a rate of 10°C/min, and then heated again to 230°C at a rate of 10°C/min to observe the temperature of the inflection point on an endothermic/exothermic curve. The temperature of the inflection point measured during the second heating was regarded as a glass transition temperature.

**Table 3**

| HPMCAS | glass transition temperature (°C) |
|---|---|
| HPMCAS-1 | 85 |
| HPMCAS-2 | 100 |
| HPMCAS-3 | 104 |
| HPMCAS-4 | 108 |
| HPMCAS-5 | 99 |
| HPMCAS-6 | 97 |
| HPMCAS-7 | 126 |

### <Examples 1 to 4 and Comparative Examples 1 to 4>

Respective injection molding compositions containing HPMCAS-1 to 4 and an injection molding composition obtained by mixing HPMCAS-5 with triethyl citrate (TEC) used as a plasticizer were dried in advance to adjust their water content, as a measurement sample, to less than 1% by weight, and were injection-molded with an injection molding system ("Haake MiniJet Pro", product of Thermo Fisher). The mold temperature and the cylinder temperature were set at 80°C and 180°C, respectively, to observe the molding property of the compositions. The injection pressure was held at 900 bar for one second and then 200 bar for 5 seconds. The mold was conical with a length of 6 cm and its bottom face had a diameter of 5 mm. The mold was installed by keeping the bottom face of the mold on the injection side. After injection, the injected composition was left to stand in the mold for 10 minutes for cooling and thus a molded product was obtained. A weight fraction of each component in the injection molding composition and evaluation of the injection molding are shown in Table 4. In Table 4, regarding evaluation criteria of the injection property, "good" means that the entire mold is filled with the injected product and "poor" means that the entire mold is not filled with the injected product and the mold has a space at the tip portion thereof.

**Table 4**

| | injection molding composition | | results of injection molding | |
|---|---|---|---|---|
| | HPMCAS (wt. parts) | plasticizer (wt. parts) | injection property | surface smoothness |
| Example 1 | HPMCAS-1 (100) | none | good | smooth appearance |
| Example 2 | HPMCAS-2 (100) | none | good | smooth appearance |
| Example 3 | HPMCAS-3 (100) | none | good | smooth appearance |
| Example 4 | HPMCAS-4 (100) | none | good | smooth appearance |
| Example 5 | HPMCAS-5 (100) | none | good | smooth appearance |
| Example 6 | HPMCAS-6 (100) | none | good | smooth appearance |
| Comp.Ex.1 | HPMCAS-7 (100) | noe | poor | - |
| Comp.Ex.2 | HPMCAS-7 (100) | triacetin (11) | good | coarse appearance with a lot of chips |
| Comp.Ex.3 | HPMCAS-7 (100) | TEC (11) | good | coarse appearance with a small amount of chips |
| Comp. Ex.4 | HPMCAS-7 (100) | TEC (25) | good | unable to be taken out because of deformation and chips |

In Examples 1 to 6 using the HPMCAS having a hydroxypropoxy molar substitution of 0.40 or more, injection could be carried out in the absence of a plasticizer and the injection molded products thus obtained had a very smooth surface. In Comparative Example 1 using the HPMCAS having a hydroxypropoxy molar substitution of less than 0.4, injection could not be carried out and failed to produce a molded product, presumably because a high viscosity during injection molding due to a high glass transition temperature made it difficult to inject the composition into the mold. In Comparative Examples 2 to 4 in which HPMCAS having a hydroxypropoxy molar substitution of less than 0.4 was used in the presence a plasticizer, an injection molded product could be obtained, but chips generated by the addition of the plasticizer deprived the product of surface smoothness and made the surface rough, presumably because the adhesion property of the plasticizer made it difficult to release the product from the mold. In particular, in Comparative Example 4 in which the composition contained 20 parts by weight of TEC based on the whole weight, injection molding could be carried out, but deformation and chips occurred during releasing of the molded product from the mold, thereby preventing the molded product from being taken out.

A tensile tester ("TENSILON:RTC-1310A", product of ORIENTEC) was used to measure the strength of the injection-molded product. The distance between pinches was adjusted to 2 cm in advance. After the injection-molded product was installed with the bottom face of the cone upside, it was clamped with the lower pinch at a position 4 cm away from the upper part thereof, and then clamped with the upper pinch above said position. The tensile speed was set at 10 mm/min and a breaking load and a breaking elongation were measured. The results are shown in Table 5. Compared with the injection-molded products obtained in Examples 1 to 6 and containing neither TEC nor triacetin as a plasticizer, the injection-molded products obtained in Comparative Examples 2 and 3 and containing TEC or triacetin exhibited a lower breaking load and a lower breaking elongation. It is considered that this occurred because addition of the plasticizer lowered the strength of the injection-molded product.

**Table 5**

| | tensile tests | |
|---|---|---|
| | breaking strength (N) | breaking elongation (mm) |
| Example 1 | 155 | 4.4 |
| Example 2 | 153 | 3.6 |
| Example 3 | 165 | 4.2 |
| Example 4 | 157 | 3.7 |
| Example 5 | 148 | 4.1 |
| Example 6 | 156 | 4.0 |
| Comp.Ex.2 | 101 | 3.5 |
| Comp.Ex.3 | 133 | 3.5 |

## Claims

1. An injection molding composition comprising hypromellose acetate succinate having a hydroxypropoxy molar substitution of 0.40 or more.

2. The injection molding composition according to Claim 1, wherein the hypromellose acetate succinate has a glass transition temperature (Tg) of 110°C or less.

3. Use of the injection molding composition claimed in Claim 1 or 2 in the manufacture of a hard capsule.

4. A method for producing an injection molded product, comprising a step of injecting into a mold the injection molding composition as claimed in Claims 1 or 2 at from 50 to 250°C.

5. The method for producing an injection molded product according to Claim 4 wherein the injection molded product is a hard capsule.
